(19) Europäisches Patentamt
European Patent Office
Office européen des brevets

(11) Numéro de publication : **0 082 781**
**B1**

# (12) FASCICULE DE BREVET EUROPÉEN

(45) Date de publication du fascicule du brevet : **03.04.85**

(21) Numéro de dépôt : **82402326.1**

(22) Date de dépôt : **17.12.82**

(51) Int. Cl.⁴ : **C 07 C 69/738**, C 07 C 67/307, C 07 C 45/54, C 07 C 45/65, C 07 C 49/203, C 07 C 49/227

(54) **Nouveaux bêta-cétoesters chlorés, leur préparation et leur emploi.**

(30) Priorité : **18.12.81 FR 8123684**

(43) Date de publication de la demande : **29.06.83 Bulletin 83/26**

(45) Mention de la délivrance du brevet : **03.04.85 Bulletin 85/14**

(84) Etats contractants désignés : **AT BE CH DE FR GB IT LI LU NL SE**

(56) Documents cités :
**US-A- 3 277 147**
**TEHAHEDRON LETTERS, no. 40, octobre 1972, pages 4067-4070, Pergamon Press (GB); P.L. STOTTER et al.: "alpha-Halocarbonyl compounds, I. An efficient preparation of alpha-bromoesters and alpha-bromoacids"**

(73) Titulaire : **RHONE-POULENC SANTE**
**Les Miroirs 18 Avenue d'Alsace**
**F-92400 Courbevoie Cedex (FR)**

(72) Inventeur : **Morel, Didier**
**19 avenue Jean Mermoz**
**F-69008 Lyon (FR)**

(74) Mandataire : **Pilard, Jacques et al**
**RHONE-POULENC RECHERCHES Service Brevets Pharma 25, Quai Paul Doumer**
**F-92408 Courbevoie Cedex (FR)**

**EP 0 082 781 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux β-cétoesters chlorés de formule générale :

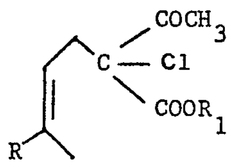

ou

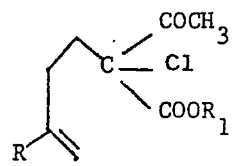

(Ia) (Ib)

dans laquelle R représente un radical alcoyle contenant 1 à 6 atomes de carbone ou un radical alcényle contenant 2 à 6 atomes de carbone, et $R_1$ représente un radical alcoyle contenant 1 à 4 atomes de carbone, leur préparation et leur emploi.

Les produits de formule générale (Ia) ou (Ib) ont un réel intérêt industriel et économique dans la mesure où ils sont utilisés comme intermédiaires pour la synthèse de vitamines ou de parfums. Plus particulièrement, le produit de formule générale (Ia) ou (Ib) dans laquelle R représente le radical méthyl-4 pentène-3 yle est un intermédiaire de la synthèse de la pseudo-ionone qui est utile pour préparer la vitamine A.

Il est connu, en particulier d'après E. W. Warnhoff et coll., Organic Syntheses, Coll. Vol. IV, 162 (1963) de préparer des α-chlorocétones par action du chlorure de sulfuryle sur la cétone correspondante, mais, lorsque la cétone est insaturée, il se produit une addition de chlore sur la double liaison. D'après E. M. Kosower et coll., J. Org. Chem. *28*, 630 et 633 (1963), l'halogénation des cétones saturées ou insaturées peut être réalisée au moyen d'un halogénure cuivrique, de préférence le chlorure cuivrique, en présence de chlorure de lithium, en opérant dans le diméthylformamide à une température comprise entre 80 et 90 °C. Cependant l'utilisation d'un tel procédé conduit généralement à l'obtention de produits polyhalogénés et les rendements ne sont pas quantitatifs.

Selon la présente invention, les produits de formule générale (Ia) ou (Ib) peuvent être obtenus par action du chlorure cuivrique, en présence de chlorure de lithium, en opérant à une température comprise entre 15 et 50 °C dans un solvant aprotique polaire basique, tel que la N-méthylpyrrolidone, le diméthylformamide, le diméthylacétamide, la tétraméthylurée ou l'hexaméthylphosphotriamide sur un produit de formule générale :

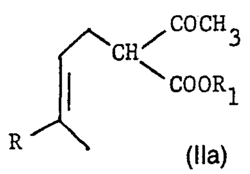

ou

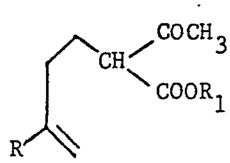

(IIa) (IIb)

dans laquelle R et $R_1$ sont définis comme précédemment. Il est particulièrement avantageux d'opérer dans la N-méthylpyrrolidone.

Généralement l'halogénation est effectuée au moyen d'un excès de chlorure cuivrique en présence d'une quantité pratiquement stœchiométrique de chlorure de lithium. De cette manière les produits de formule générale (Ia) ou (Ib) sont obtenus avec des rendements pratiquement quantitatifs et sans formation de produits polyhalogénés.

Les produits de formule générale (IIa) ou (IIb) peuvent être obtenus par addition sélective d'un cétoester de formule générale :

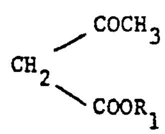

(III)

dans laquelle $R_1$ est défini comme précédemment sur un butadiène de formule générale :

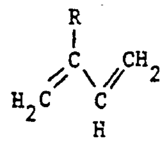

(IV)

dans laquelle R est défini comme précédemment.

Généralement la réaction est effectuée dans l'eau ou dans un milieu hydroalcoolique contenant au maximum 50 % d'un alcool aliphatique contenant 1 à 3 atomes de carbone en présence d'un catalyseur constitué, d'une part, par au moins une phosphine soluble dans l'eau et, d'autre part, par au moins un composé d'un métal de transition, le catalyseur étant en solution dans l'eau et le composé du métal de transition étant un composé de rhodium.

Les phosphines solubles dans l'eau sont celles décrites dans le brevet français 76 22824 et plus particulièrement celles qui répondent à la formule :

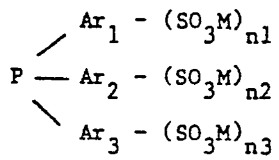

(V)

dans laquelle

$Ar_1$, $Ar_2$ et $Ar_3$, identiques ou différents, représentent chacun un radical choisi parmi le groupe comprenant les radicaux phénylène et les radicaux naphtylène éventuellement substitués,

M est un reste cationique d'origine minérale ou organique choisi de manière que la phosphine de formule (V) soit soluble dans l'eau,

n1, n2 et n3, identiques ou différents, sont des nombres entiers supérieurs et égaux à 0 et inférieurs ou égaux à 3, l'un au moins étant supérieur ou égal à 1.

Le composé du rhodium utilisé doit être soluble dans l'eau ou capable de passer en solution dans l'eau dans les conditions de la réaction par réaction de coordination avec les phosphines hydrosolubles. D'un intérêt tout particulier sont $RhCl_3$ et $[RhCl\ (cyclooctadiène\text{-}1,5)]_2$

On utilise une quantité de composé du rhodium telle que le nombre d'atomes-gramme de rhodium élémentaire par litre de solution réactionnelle soit compris entre $10^{-4}$ et 1.

La quantité de phosphine est choisie de telle manière que le nombre d'atomes-gramme de phosphine trivalent rapporté à un atome-gramme de rhodium soit compris entre 0,1 et 200.

La quantité d'eau minimum est celle qui est suffisante pour dissoudre le catalyseur en totalité et au moins une partie des réactifs.

La température de la réaction est généralement inférieure à 200 °C et, de préférence, est comprise entre 50 °C et 125 °C.

Il est particulièrement avantageux d'opérer en présence d'un excès du produit de formule générale (III) par rapport au produit de formule générale (IV).

Pour améliorer la réactivité, il est possible d'ajouter au milieu réactionnel une base minérale (hydroxydes, carbonates, bicarbonates de métaux alcalins ou alcalino-terreux) ou organique (amines tertiaires aliphatiques ou aromatiques) à une concentration comprise entre 0,005 et 5 moles de base par litre de solution aqueuse.

Les produits de formule générale (Ia) ou (Ib) sont particulièrement utiles pour préparer les cétones éthyléniques de formule générale :

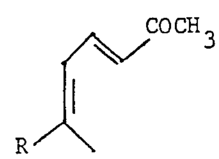

(VI)

dans laquelle R est défini comme précédemment, sous forme d'un mélange des isomères EE ou EZ.

Le procédé de préparation des produits de formule générale (VI) consiste à décarboxyler le produit de formule générale (Ia) ou (Ib) pour obtenir l'α-chlorocétone de formule générale :

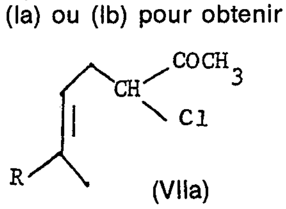

(VIIa)

ou

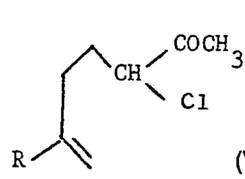

(VIIb)

dans laquelle R est défini comme précédemment qui est déhydrohalogénée pour obtenir le produit de formule générale (VI).

Selon la présente invention, les réactions de décarboxylation et de déhydrohalogénation peuvent être effectuées sans isolement de l'α-chlorocétone de formule générale (VIIa) ou (VIIb).

Il est connu, en particulier d'après A. P. Krapcho et coll., J. Org. Chem., *43* (1) 138 (1978), de décarboxyler les gem-diesters, les β-cétoesters ou les α-cyanoesters en présence d'un excès de sel

minéral, tel que le chlorure de lithium, en opérant dans le diméthylsulfoxyde aqueux à la température de reflux du mélange réactionnel.

Par ailleurs, il est connu, en particulier d'après E. W. Warnhoff et coll., Organic Syntheses, Coll. Vol. IV, 162 (1963) de déhydrohalogéner une α-halocétone en présence de chlorure de lithium dans le diméthylformamide à une température voisine de 100 °C.

Il a maintenant été trouvé que les α-chloro β-cétoesters de formule générale (I) conduisent aux α-chlorocétones de formule générale (VIIa) ou (VIIb), avec des rendements pratiquement quantitatifs, par traitement au moyen de chlorure de lithium dans un solvant aprotique polaire basique, tel que la N-méthylpyrrolidone, la diméthylformamide, la diméthylacétamide, la tétraméthylurée ou l'hexaméthylphosphotriamide (de préférence la N-méthylpyrrolidone), à une température voisine de 130 °C, en présence d'eau ou d'un acide minéral fort tel que l'acide chlorhydrique ou l'acide sulfurique. Il est particulièrement avantageux d'opérer en présence d'un acide minéral fort afin d'éviter la réaction secondaire de déacylation.

Généralement, la réaction est effectuée en présence d'une quantité molaire de chlorure de lithium représentant 1 à 5 fois environ la quantité molaire du produit de formule générale (Ia) ou (Ib). De préférence, on utilise une quantité de chlorure de lithium voisine de la stœchiométrie.

La quantité d'eau ou d'acide minéral est voisine de la stœchiométrie.

Selon la composition du mélange réactionnel la décarboxylation est complète après un temps de chauffage compris entre 10 minutes et 5 heures à une température voisine de 100 °C.

Les α-chlorocétones de formule générale (VIIa) ou (VIIb) sont transformées en cétones éthyléniques de formule générale (VI) de préférence par chauffage en présence de chlorure de lithium dans un solvant aprotique polaire basique, tel que la N-méthylpyrrolidone, le diméthylformamide, le diméthylacétamide ou l'hexaméthylphosphotriamide, (de préférence la N-méthylpyrrolidone) éventuellement en présence d'une amine tertiaire très basique telle que la triméthyl-2,4,6 pyridine (collidine) ou l'éthyldicyclohexylamine. La quantité molaire de l'amine tertiaire représente environ 1 à 5 fois la quantité molaire du produit de formule générale (Ia) ou (Ib). De préférence, on utilise une quantité d'amine tertiaire voisine du double de la stoechiométrie. La réaction de déhydrohalogénation est généralement complète après un chauffage à une température comprise entre 80 et 160 °C pendant 1 à 20 heures.

Pour la réalisation plus commode du procédé selon l'invention, il est particulièrement avantageux d'effectuer les réactions de décarboxylation et de déhydrohalogénation sans isolement intermédiaire de l'α-chlorocétone de formule générale (VIIa) ou (VIIb) en utilisant le système chlorure de lithium-acide minéral-amine tertiaire en solution dans la N-méthylpyrrolidone à une température comprise entre 80 et 160 °C pendant 1 à 20 heures.

Les cétones de formule générale (VI) obtenues selon le procédé de la présente invention peuvent être éventuellement purifiées selon les méthodes physico-chimiques telles que la distillation ou la chromatographie.

Les exemples suivants, donnés à titre non limitatif, illustrent la présente invention.

Exemple 1 — Chloration du mélange de ($IIa_1$) et de ($IIb_1$)

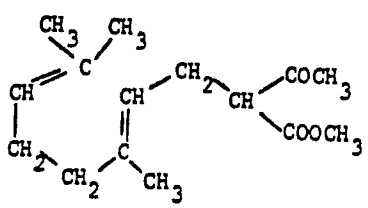

($IIa_1$) ; 48 %

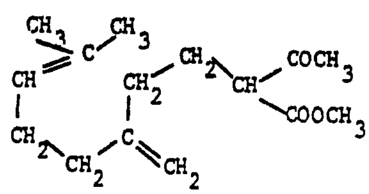

($IIb_1$) ; 52 %

A un mélange de 32,3 g de chlorure cuivrique (0,24 mole) et de 5,1 g de chlorure de lithium (0,12 mole) dans 100 $cm^3$ de N-méthylpyrrolidone, on ajoute 24,66 g du mélange de ($IIa_1$) et de ($IIb_1$) (0,102 mole). On maintient le mélange réactionnel à 20 °C pendant 91 heures. Le mélange réactionnel est alors dilué par addition de 600 $cm^3$ d'eau. Le chlorure cuivreux qui a précipité est séparé par filtration puis le mélange réactionnel est extrait par 2 fois 50 $cm^3$ de pentane. Après séchage des couches organiques sur sulfate de sodium et concentration sous pression réduite, on obtient 28,39 g du mélange de ($Ia_1$) et de ($Ib_1$) dont la pureté est voisine de 95 %, et qui présente les caractéristiques suivantes :

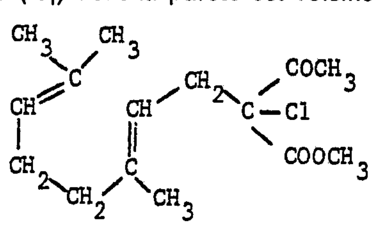

($Ia_1$) ; 45 %

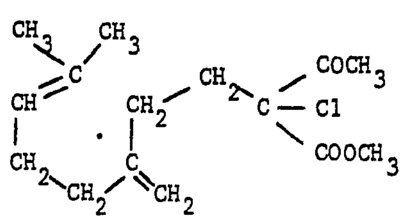

($Ib_1$) ; 55 %

P.E.$_{0,027\ kPa}$ (mélange) = 120 °C

teneur en chlore du mélange = 12,4 %

Le taux de transformation du mélange de ($IIa_1$) et de ($IIb_1$) est égal à 95 % ; la sélectivité est égale à 100 %.

Le mélange des produits de formule ($IIa_1$) et ($IIb_1$) peut être préparé de la façon suivante :

Dans un réacteur en acier inoxydable préalablement purgé à l'argon, on introduit 40,8 mg de [RhCl(cyclooctadiène-1,5)]$_2$
(0,165 milliatome-gramme de rhodium), 2,2 g de

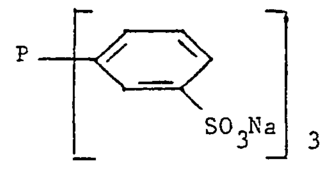

(ou TPPTSNa)

(3,256 milliatome-gramme de $P^{3+}$), 78,2 mg de carbonate de sodium
(0,68 millimole), 15 cm$^3$ d'eau et 5 cm$^3$ de méthanol. On introduit ensuite 15,92 g de myrcène (0,117 mole) et 13,9 g d'acétylacétate de méthyle (0,120 mole).

On chauffe sous agitation à 90 °C pendant 1 heure.

Après traitement du mélange réactionnel, on obtient 12,488 g du mélange des produits de formule ($IIa_1$) et ($IIb_1$).

Exemple 2 — Chloration du mélange de ($IIa_2$) et de ($IIb_2$)

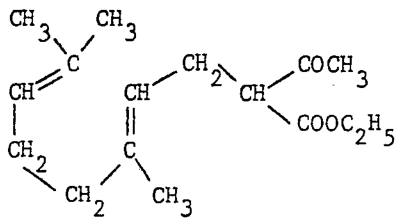

($IIa_2$) ; 45 %

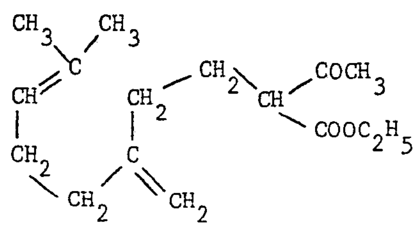

($IIb_2$) ; 55 %

A un mélange de 120 g de chlorure cuivrique (0,89 mole) et de 17 g de chlorure de lithium (0,4 mole) dans 300 cm$^3$ de N-méthylpyrrolidone, on ajoute 107 g du mélange des produits de formule ($IIa_2$) et ($IIb_2$) (0,402 mole). On obtient le mélange réactionnel à 20 °C pendant 90 heures. Le mélange réactionnel est alors dilué par addition de 2 litres d'eau froide. Le chlorure cuivreux qui a précipité est séparé par filtration puis le mélange réactionnel est extrait par 2 fois 200 cm$^3$ de pentane. Après séchage des couches organiques sur sulfate de sodium et concentration sous pression réduite, on obtient 119 g du mélange des produits des formules ($Ia_2$) et ($Ib_2$), dont la pureté est voisine de 92 % et qui présente les caractéristiques suivantes :

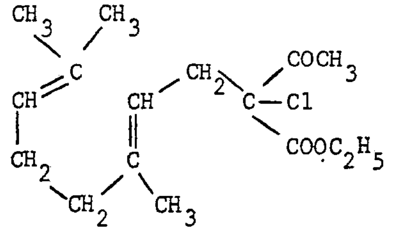

($Ia_2$) ; 45 %

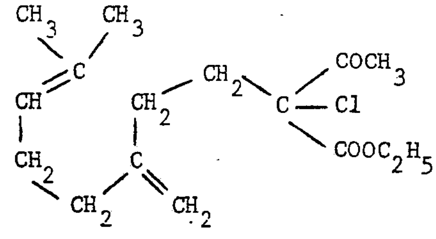

($Ib_2$) ; 55 %

P.E.$_{0,027\ kPa}$ (mélange) = 129 °C

teneur en chlore (mélange) = 11,8 %

Le taux de transformation du mélange de ($IIa_2$) et de ($IIb_2$) est égal à 91 % ; la sélectivité en ($Ia_2$) et ($Ib_2$) est égale à 100 %.

Le mélange des produits ($IIa_2$) et ($IIb_2$) peut être préparé de la façon suivante :

Dans un réacteur en acier inoxydable préalablement purgé à l'argon, on introduit 100 mg de [RhCl(cyclooctadiène-1,5)]$_2$ (0,41 milliatome-gramme de rhodium), 0,78 g de TPPTS Na (1,15 milliatome-gramme de $P^{3+}$), 0,156 g de carbonate de sodium (1,5 millimole) et 30 cm$^3$ d'eau. On introduit ensuite 8,8 g de myrcène (64,7 millimoles) et 16,5 g d'acétylacétate d'éthyle (127 millimoles). On chauffe sous agitation à 100 °C pendant 6 heures. Après traitement du mélange réactionnel, on obtient 12,66 g du mélange de ($IIa_2$) et de ($IIb_2$).

Exemple 3 — Chloration du mélange de ($IIa_3$) et de ($IIb_3$)

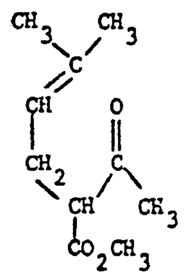

($IIa_3$) ; 40 %

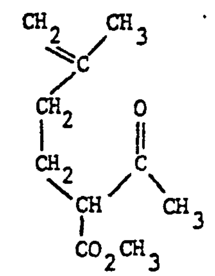

($IIb_3$) ; 60 %

A un mélange de 32,3 g de chlorure cuivrique (0,24 mole) et de 5,1 g de chlorure de lithium (0,12 mole) dans 100 cm$^3$ de N-méthylpyrrolidone, on ajoute 18,87 g du mélange des produits ($IIa_3$) et ($IIb_3$) (0,102 mole). On maintient le mélange réactionnel à 20 °C pendant 27 heures. Après traitement du mélange réactionnel comme dans l'exemple 1, on obtient 21,57 g du mélange de ($Ia_3$) et de ($Ib_3$), dont la pureté est voisine de 88 % et qui présente les caractéristiques suivantes :

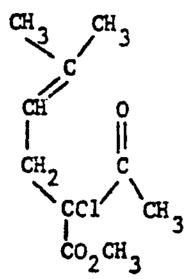

($Ia_3$) ; 40 %

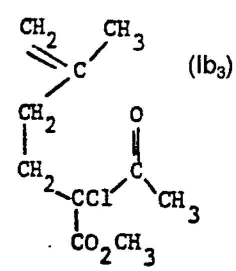

($Ib_3$) ; 60 %

P.E.$_{0,067\ kPa}$ (mélange) = 64 °C

teneur en chlore (mélange) : 16,2 %

Le taux de transformation du mélange de ($IIa_3$) et de ($IIb_3$) est égal à 88 % ; la sélectivité en ($Ia_3$) et ($Ib_3$) est égale à 100 %.

Le mélange de ($IIa_3$) et de ($IIb_3$) peut être préparé de la manière suivante :

Dans un réacteur en acier inoxydable préalablement purgé à l'argon, on introduit 20,8 mg de [RhCl(cyclooctadiène-1,5)]$_2$ (0,085 milliatome-gramme de rhodium), 0,24 g de carbonate de sodium (2,2 millimoles), 1,128 g de TPPTSNa (1,7 milliatome-gramme de $P^{3+}$) et 15 cm$^3$ d'eau. On introduit ensuite 10,2 g d'isoprène (150 millimoles) et 21,6 g d'acétylacétate de méthyle (186 millimoles). On chauffe sous agitation à 100 °C pendant 1 heure. Après traitement du mélange réactionnel on obtient 15,85 g du mélange de ($IIa_3$) et de ($IIb_3$).

Exemple 4 — Chloration du mélange de ($IIa_1$) et de ($IIb_1$)

A un mélange de 5,38 g de chlorure cuivrique (0,40 mole) et 0,87 g de chlorure de lithium (0,020 mole) dans 20 cm$^3$ de diméthylformamide, on ajoute 5,1 g du mélange de ($IIa_1$) et de ($IIb_1$) (0,020 mole). On maintient le mélange à 20 °C pendant 70 heures. Après traitement du mélange réactionnel comme dans l'exemple 1, on obtient 5,26 g du mélange de ($Ia_1$) et de ($Ib_1$), dont la pureté est voisine de 60 % et qui présente les caractéristiques suivantes :

($Ia_1$) ; 45 % ($Ib_1$) ; 55 %

Le taux de transformation du mélange de ($IIa_1$) et de ($IIb_1$) est égal à 62 % ; la sélectivité en ($Ia_1$) et ($Ib_1$) est égale à 100 %.

Exemple 5

A 1 g de chlorure de lithium (0,023 5 mole) dans 10 cm$^3$ de N-méthylpyrrolidone et 0,18 g d'eau (0,010

mole), on ajoute 2,56 g du mélange de ($Ia_1$) et de ($Ib_1$) obtenu à l'exemple 1 (8,5 moles). Le mélange est maintenu sous une atmosphère inerte d'argon puis il est chauffé à 100 °C pendant 45 minutes sous agitation ; la décarboxylation est alors totale. Le chauffage est ensuite poursuivi pendant 17 heures à la même température. Après traitement du mélange réactionnel par addition de 30 $cm^3$ d'eau, extraction des produits avec 20 $cm^3$ de pentane et élimination du pentane sous pression réduite, on obtient 1,83 g d'un mélange contenant, d'après l'analyse par chromatographie en phase gazeuse (CPG) 0,559 g de pseudo-ionone, 0,34 g du mélange des produits de formule ($VIIa_1$) et ($VIIb_1$) dans laquelle R est le radical méthyl-4 pentène-3 yle et 0,6 g de produits lourds non élués en CPG.

Le taux de déshydrochloration de (VII) est égal à 82 % et le rendement en (VI) égal à 41,5 %.

Exemple 6

A 0,46 g de chlorure de lithium (0,010 8 mole) dans 5 $cm^3$ de N-méthylpyrrolidone et 0,3 g d'eau (0,016 6 mole), on ajoute 3,14 g du mélange de ($Ia_1$) et de ($Ib_1$) obtenu à l'exemple 1 (0,011 mole). Le mélange est chauffé à 105 °C pendant 40 minutes. Le mélange réactionnel contient alors 1,87 g (0,008 2 mole) d'un mélange de ($VIIa_1$) et de ($VIIb_1$)

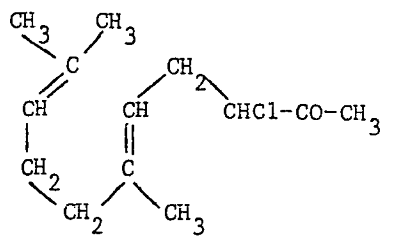

($VIIa_1$) ; 45 %

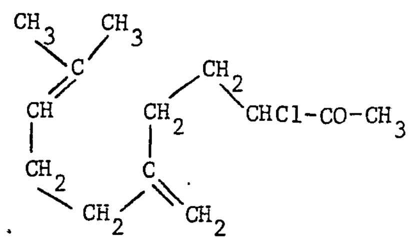

($VIIb_1$) ; 55 %

P.E.$_{0,054\ kPa}$ (mélange) = 93 °C
Le taux de déacylation est voisin de 25 %.

Exemple 7

A 0,5 g de chlorure de lithium (11,7 millimoles) dans 5 $cm^3$ de N-méthylpyrrolidone contenant 0,49 g d'acide chlorhydrique (13,4 millimoles), on ajoute 2,64 g du mélange de ($Ia_1$) et de ($Ib_1$) obtenu à l'exemple 1 (9,2 millimoles). Le mélange est chauffé à 105 °C pendant 2 heures 30 minutes. Après traitement du mélange réactionnel, on obtient 1,025 g du mélange de ($VIIa_1$) et de ($VIIb_1$) (4,5 millimoles). Il y a formation de produits de dégradation et le taux de déacylation est voisin de 5 %.

Exemple 8

A 0,5 g de chlorure de lithium (11,7 millimoles) dans 5 $cm^3$ de N-méthylpyrrolidone contenant 0,13 g d'acide chlorhydrique (3,6 millimoles), on ajoute 2,64 g du mélange de ($Ia_1$) et de ($Ib_1$) obtenu à l'exemple 1 (9,2 millimoles). Le mélange est chauffé à 130 °C sous agitation pendant 2 heures. La décarboxylation est totale en 15 minutes. Après traitement du mélange réactionnel, on obtient, avec un rendement de 40,5 %, 0,716 g de pseudo-ionone. Le taux de déacylation est voisin de 5 %. Il se forme environ 28 % de produits lourds non élués en CPG.

La sélectivité en pseudo-ionone est égale à 57,4 % pour un taux de déhydrochloration égal à 70,6 %.

Exemple 9

A 5,0 g de chlorure de lithium (11,7 millimoles) dans 5 $cm^3$ de N-méthylpyrrolidone contenant 0,19 g d'acide chlorhydrique (5,7 millimoles), on ajoute 2,22 g du mélange de ($Ia_1$) et de ($Ib_1$) obtenu à l'exemple 1 (9,5 millimoles). Le mélange est maintenu sous atmosphère d'argon, puis il est chauffé à 140 °C pendant 1 heure sous agitation. La décarboxylation est totale en 15 minutes.

Après traitement du mélange réactionnel, on obtient, avec un rendement de 40 %, 0,734 g de pseudo-ionone. Le taux de déacylation est voisin de 5 %. Il se forme environ 18 % de produits lourds non élués en CPG.

La sélectivité en pseudo-ionone est égale à 60,6 % pour un taux de déhydrohalogénation de 66 %.

Exemple 10

A 0,5 g de chlorure de lithium (11,7 millimoles) dans 5 $cm^3$ de N-méthylpyrrolidone contenant 0,44 g

d'acide chlorhydrique (12 millimoles), on ajoute 1,27 g de triméthyl-2,4,6 pyridine (10,5 millimoles) et 2,58 g du mélange de ($Ia_1$) et de ($Ib_1$) obtenu à l'exemple 1 (9 millimoles). Le mélange est alors chauffé à 130 °C pendant 4 heures, sous agitation. La décarboxylation est complète en 15 minutes. Après traitement du mélange réactionnel, on obtient, avec un rendement de 52,5 %, 0,909 g de pseudo-ionone. Le taux de déacylation est inférieur à 1 %. Il se forme environ 2,5 % de produits lourds non élués en CPG.

La sélectivité en pseudo-ionone est égale à 90 % pour un taux de déhydrochloration de 58,1 %.

Exemple 11

A 0,5 g de chlorure de lithium (11,7 millimoles) dans 10 $cm^3$ de N-méthylpyrrolidone contenant 0,46 g d'acide chlorhydrique (12,6 millimoles), on ajoute 1,26 g de triméthyl-2,4,6 pyridine (10,5 millimoles) et 2,70 g du mélange de ($Ia_1$) et de ($Ib_1$) obtenu à l'exemple 1 (9,4 millimoles). Le mélange est alors chauffé à 130 °C pendant 8 heures, sous agitation. La décarboxylation est complète en 15 minutes. Après traitement du mélange réactionnel, on obtient, avec un rendement de 59,7 %, 1,069 g de pseudo-ionone.

Le taux de déacylation est inférieur à 1 %. Il se forme environ 12 % de produits lourds non élués en CPG.

La sélectivité en pseudo-ionone est égale à 62,5 % pour un taux de déhydrochloration de 95,50 %.

Exemple 12

On opère comme dans l'exemple 11, mais en utilisant 20 $cm^3$ de N-méthylpyrrolidone.

On obtient, avec un rendement de 69,7 %, 1,274 g de pseudo-ionone.

Le taux de déacylation est inférieur à 1 %. Il se forme environ 9 % de produits lourds non élués en CPG.

La sélectivité en pseudo-ionone est égale à 75,5 % pour un taux de déhydrochloration de 92,4 %.

Exemple 13

A 0,5 g de chlorure de lithium (11,7 millimoles) dans 20 $cm^3$ de N-méthylpyrrolidone contenant 0,45 g d'acide chlorhydrique (12,3 millimoles), on ajoute 2,70 g du mélange de ($Ia_1$) et de ($Ib_1$) obtenu à l'exemple 1 (9,4 millimoles). Le mélange est alors chauffé à 150 °C pendant 2 heures 30 minutes, sous agitation. La décarboxylation est complète en 10 minutes. Après traitement du mélange réactionnel, on obtient, avec un rendement de 28,2 %, 0,509 g de pseudo-ionone.

Le taux de déacylation est inférieur à 1 %. Il se forme environ 10,5 % de produits lourds non élués en CPG.

La sélectivité en pseudo-ionone est égale à 29,3 % pour un taux de déhydrochloration de 96,2 %.

Exemple 14

On opère comme dans l'exemple 13, mais en ajoutant 1,26 g de triméthyl-2,4,6 pyridine (10,5 millimoles) et 2,55 g du mélange de ($Ia_1$) et de ($Ib_1$) obtenu à l'exemple 1 (8,9 millimoles). On obtient, avec un rendement de 71,2 %, 1,218 g de pseudo-ionone.

Le taux de déacylation est inférieur à 1 %. Il se forme environ 11 % de produits lourds non élués en CPG.

La sélectivité en pseudo-ionone est égale à 73,2 % pour un taux de déhydrochloration de 97,2 %.

Exemple 15

On opère comme dans l'exemple 13, mais en ajoutant 2,83 g de triméthyl-2,4,6 pyridine (23,3 millimoles) et 2,66 g du mélange de ($Ia_1$) et de ($Ib_1$) obtenu à l'exemple 1 (9,3 millimoles). On obtient, avec un rendement de 76,2 %, 1,366 g de pseudo-ionone.

Le taux de déacylation est inférieur à 1 %. Il se forme environ 12,5 % de produits lourds non élués en CPG.

La sélectivité en pseudo-ionone est égale à 79 % pour un taux de déhydrochloration de 96,5 %.

Exemple 16

On opère comme dans l'exemple 13, mais en ajoutant 4,25 g d'éthyldicyclohexylamine (22,7 millimoles) et 2,66 g du mélange de ($Ia_1$) et de ($Ib_1$) obtenu à l'exemple 1 (9,3 millimoles).

On obtient, avec un rendement de 77,4 %, 1,385 g de pseudo-ionone.

Le taux de déacylation est inférieur à 1 %. Il se forme environ 1 % de produits lourds non élués en CPG.

La sélectivité en pseudo-ionone est égale à 85,7 % pour un taux de déhydrochloration de 90,4 %.

Exemple 17

A 0,5 g de chlorure de lithium (11,7 millimoles) dans 20 $cm^3$ de N-méthylpyrrolidone, on ajoute 1,29 g

de triméthyl-2,4,6 pyridin (10,6 millimoles) et 1,94 g du mélange de ($VIIa_1$) et de ($VIIb_1$) obtenu à l'exemple 6 (8,5 millimoles). Le mélange est chauffé à 150 °C. On obtient, avec un rendement de 88 %, 1,44 g de pseudo-ionone.

Il se forme environ 6,5 % de produits lourds non élués en CPG.

La sélectivité en pseudo-ionone est égale à 94,6 % pour un taux de déhydrochloration de 93 %.

Exemple 18

On opère comme dans l'exemple 13, mais en ajoutant 4,82 g d'éthyldicyclohexylamine (23 millimoles) et 2,70 g du mélange de ($Ia_2$) et de ($Ib_2$) obtenu à l'exemple 2 (9 millimoles).

On obtient, avec un rendement de 79,9 %, 1,377 g de pseudo-ionone.

Le taux de déacylation est inférieur à 1 %. Il se forme environ 2 % de produits lourds non élués en CPG.

La sélectivité en pseudo-ionone est égale à 90,2 % pour un taux de déhydrochloration de 88,6 %.

Exemple 19

A 0,5 g de chlorure de lithium (11,7 millimoles) dans 20 $cm^3$ de tétraméthylurée contenant 0,5 g d'acide chlorhydrique (13,7 millimoles), on ajoute 2,72 g du mélange de ($Ia_1$) et de ($Ib_1$) obtenu à l'exemple 1 (9,5 millimoles). Le mélange est alors chauffé à 150 °C pendant 2 heures 30 minutes, sous agitation. La décarboxylation est complète en 15 minutes. Après traitement du mélange réactionnel, on obtient, avec un rendement de 55,9 %, 1,0 g de pseudo-ionone.

Le taux de déacylation est inférieur à 1 %. Il se forme environ 5 % de produits lourds non élués en CPG.

La sélectivité en pseudo-ionone est égale à 80 % pour un taux de déhydrochloration de 68,4 %.

Exemple 20

A 6,45 g de chlorure de lithium (0,15 mole) dans 250 $cm^3$ de N-méthylpyrrolidone contenant 5,77 g d'acide chlorhydrique (0,16 mole), on ajoute 64,52 g d'éthyldicyclohexylamine (0,31 mole) et 32,77 g du mélange des produits ($Ia_3$) et de ($Ib_3$) obtenu à l'exemple 3 (0,15 mole). Le mélange est alors chauffé à 150 °C pendant 3 heures 30 minutes sous agitation. La décarboxylation est complète en 10 minutes. Après traitement du mélange réactionnel, on obtient, avec un rendement de 75,5 %, 14,237 g de méthylheptadiènone de formule ($VIa_2$)

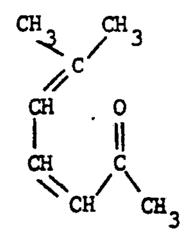

($VIa_2$)

Le taux de déacylation est inférieur à 1 %. Il se forme environ 7 % de produits lourds non élués en CPG.

La sélectivité en méthylheptadiènone de formule ($VIa_2$) est égale à 70 % pour un taux de déhydrochloration égal à 95,6 %.

Exemple 21

A 0,5 g de chlorure de lithium (11,7 millimoles) dans 20 $cm^3$ de diméthylformamide contenant 0,42 g d'acide chlorhydrique (11,5 millimoles), on ajoute 4,18 g d'éthyldicyclohexylamine (20 millimoles) et 2,72 g du mélange des produits ($Ia_1$) et ($Ib_1$) obtenu à l'exemple 1 (9,5 millimoles). Le mélange est alors chauffé à 150 °C pendant 2 heures 30 minutes sous agitation. La décarboxylation est complète en 15 minutes. Après traitement du mélange réactionnel, on obtient, avec un rendement de 59,3 %, 1,079 g de pseudo-ionone.

Le taux de déacylation est inférieur à 1 %. Il se forme environ 6,5 % de produits lourds non élués en CPG.

La sélectivité en pseudo-ionone est égale à 80 % pour un taux de déhydrochloration égal à 74,1 %.

**Revendications** (pour les Etats contractants : BE, CH, DE, FR, GB, IT, LI, LU, NL, SE)

1. Un β-cétoester chloré caractérisé en ce qu'il répond à la formule générale :

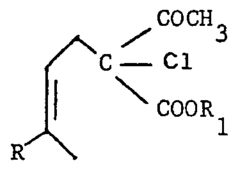

ou

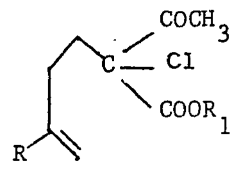

(Ia) (Ib)

dans laquelle R représente un radical alcoyle contenant 1 à 6 atomes de carbone ou un radical aldényle contenant 2 à 6 atomes de carbone, et $R_1$ représente un radical alcoyle contenant 1 à 4 atomes de carbone.

2. Un procédé de préparation d'un produit selon la revendication 1 caractérisé en ce que l'on fait réagir le chlorure cuivrique en présence de chlorure de lithium dans un solvant aprotique polaire basique à une température comprise entre 15 et 50 °C, sur un produit de formule générale :

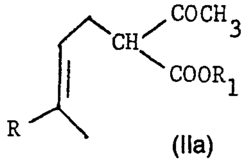

(IIa)

ou

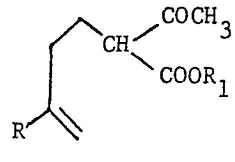

(IIb)

dans laquelle R et $R_1$ sont définis comme dans la revendication 1.

3. Un procédé selon la revendication 1 caractérisé en ce que le solvant aprotique polaire basique est choisi parmi la N-méthylpyrrolidone, la diméthylformamide, le diméthylacétamide, la tétraméthylurée et l'hexaméthylphosphotriamide.

4. Utilisation d'un produit selon la revendication 1 pour la préparation d'une cétone éthylénique de formule générale :

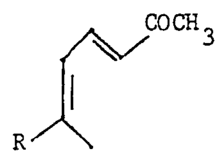

dans laquelle R est défini comme dans la revendication 1 caractérisée en ce qu'un produit selon la revendication 1 est soumis à l'action du chlorure de lithium en présence d'eau ou d'un acide minéral dans un solvant aprotique polaire basique à une température voisine de 100 °C pour donner une α-chlorocétone de formule générale :

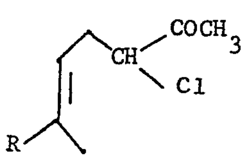

ou

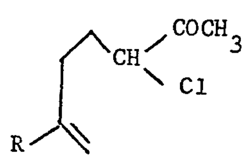

dans laquelle R est défini comme dans la revendication 1, qui est traitée par le chlorure de lithium dans un solvant aprotique polaire basique à une température comprise entre 80 et 160 °C éventuellement en présence d'une amine tertiaire très basique telle que la triméthyl-2,4,6 pyridine ou l'éthyldicyclohexylamine, pour donner la cétone éthylénique qui est isolée.

5. Utilisation d'un produit selon la revendication 1 pour la préparation d'une cétone éthylénique de formule générale :

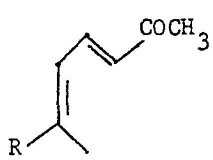

dans laquelle R est défini comme dans la revendication 1 caractérisée en ce que l'on traite un produit selon la revendication 1 par le système chlorure de lithium-acide minéral-amine tertiaire dans un solvant aprotique polaire basique, à une température comprise entre 80 °C et 160 °C, et isole le produit obtenu.

6. Procédé selon la revendication 4 ou 5 caractérisé en ce que le solvant aprotique polaire basique

est choisi parmi la N-méthylpyrrolidone, le diméthylformamide, le diméthylacétamide, la tétraméthylurée ou l'hexaméthylphosphotriamide.

**Revendications** (pour l'Etat contractant AT)

1. Procédé de préparation d'un nouveau β-cétoester chloré de formule générale :

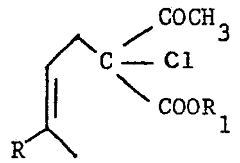

ou

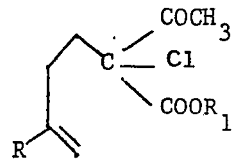

(Ia) (Ib)

dans laquelle R représente un radical alcoyle contenant 1 à 6 atomes de carbone ou un radical alcényle contenant 2 à 6 atomes de carbone et $R_1$ représente un radical alcoyle contenant 1 à 4 atomes de carbone, caractérisé en ce que l'on fait réagir le chlorure cuivrique en présence de chlorure de lithium dans un solvant aprotique polaire basique à une température comprise entre 15 et 20 °C, sur un produit de formule générale :

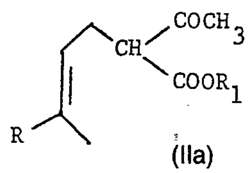

(IIa)

ou

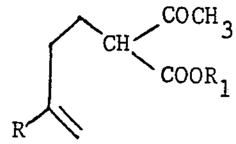

(IIb)

dans laquelle R et $R_1$ sont définis comme précédemment.

2. Procédé selon la revendication 1 caractérisé en ce que le solvant aprotique polaire basique est choisi parmi la N-méthylpyrrolidone, le diméthylformamide, le diméthylacétamide, le tétraméthylurée et l'hexaméthylphosphotriamide.

3. Utilisation d'un produit obtenu selon le procédé de la revendication 1 pour la préparation d'une cétone éthylénique de formule générale :

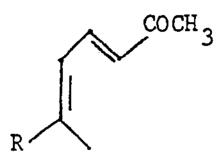

dans laquelle R est défini comme dans la revendication 1, caractérisé en ce qu'un produit obtenu selon le procédé de la revendication 1 est soumis à l'action du chlorure de lithium en présence d'eau ou d'un acide minéral dans un solvant aprotique polaire basique à une température voisine de 100 °C pour donner une α-chlorocétone de formule générale :

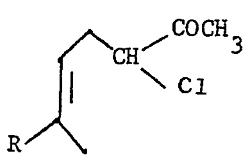

ou

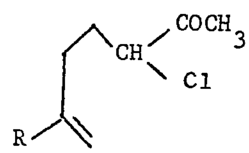

dans laquelle R est défini dans la revendication 1, qui est traitée par le chlorure de lithium dans un solvant aprotique polaire basique à une température comprise entre 80 et 160 °C éventuellement en présence d'une amine tertiaire très basique telle que la triméthyl-2,4,6 pyridine ou l'éthyldicyclohexylamine, pour donner la cétone éthylénique qui est isolée.

4. Utilisation d'un produit obtenu selon le procédé de la revendication 1 pour la préparation d'une cétone éthylénique de formule générale :

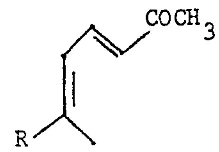

dans laquelle R est défini comme dans la revendication 1 caractérisée en ce que l'on traite un produit obtenu selon le procédé de la revendication 1 par le système chlorure de lithium-acide minéralamine tertiaire dans un solvant aprotique polaire basique, à une température comprise entre 80 et 160 °C, et isole le produit obtenu.

5. Utilisation selon la revendication 4 ou 5 caractérisée en ce que le solvant aprotique polaire basique est choisi parmi la N-méthylpyrrolidone, le diméthylformamide, le diméthylacétamide, la tétraméthylurée ou l'hexaméthylphosphotriamide.

**Claims** (for the Contracting States : BE, CH, DE, FR, GB, IT, LI, LU, NL, SE)

1. A chlorinated β-keto-ester, characterised in that it corresponds to the general formula :

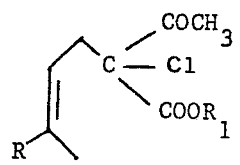

or

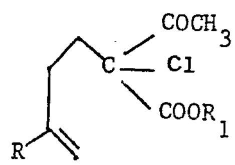

(Ia) (Ib)

in which R represents an alkyl radical containing 1 to 6 carbon atoms or an alkenyl radical containing 2 to 6 carbon atoms and $R_1$ represents an alkyl radical containing 1 to 4 carbon atoms.

2. A process for the preparation of a product according to Claim 1, characterised in that cupric chloride, in the presence of lithium chloride, is reacted, in a basic polar aprotic solvent at a temperature of between 15 and 50 °C, with a product of the general formula :

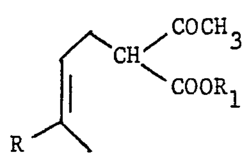

or

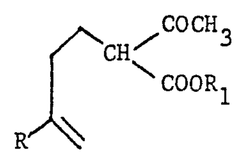

(IIa) (IIb)

in which R and $R_1$ are defined as in Claim 1.

3. A process according to Claim 1, characterised in that the basic polar aprotic solvent is chosen from among N-methylpyrrolidone, dimethylformamide, dimethylacetamide, tetramethylurea and hexamethylphosphotriamide.

4. Use of a product according to Claim 1 for the preparation of an ethylenic ketone of the general formula :

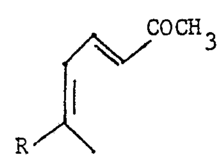

in which R is defined as in Claim 1, characterised in that a product according to Claim 1 is subjected to the action of lithium chloride in the presence of water or of a mineral acid in a basic polar aprotic solvent at a temperature of about 100 °C, to give an α-chloro ketone of the general formula :

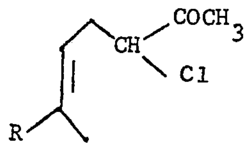

or

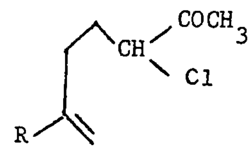

in which R is defined as in Claim 1, which compound is treated with lithium chloride in a basic polar aprotic solvent at a temperature of between 80 and 160 °C, if appropriate in the presence of a highly basic tertiary amine such as 2,4,6-trimethyl-pyridine or ethyldicyclohexylamine, to give the ethylenic ketone, which is isolated.

5. Use of a product according to Claim 1 for the preparation of an ethylenic ketone of the general formula :

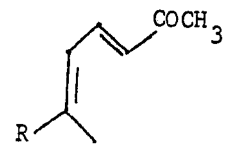

in which R is defined as in Claim 1, characterised in that a product according to Claim 1 is treated with the lithium chloride/mineral acid/tertiary amine system in a basic polar aprotic solvent at a temperature of between 80 °C and 160 °C and the product obtained is isolated.

6. Process according to Claim 4 or 5, characterised in that the basic polar aprotic solvent is chosen from among N-methylpyrrolidone, dimethylformamide, dimethylacetamide, tetramethylurea or hexamethylphosphotriamide.

**Claims** (for the Contracting State AT)

1. Process for the preparation of a novel chlorinated β-keto-ester of the general formula :

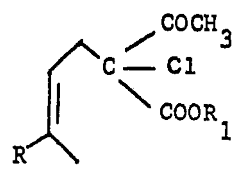

(Ia)

or

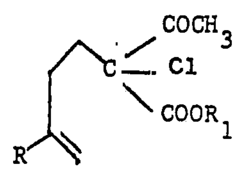

(Ib)

in which R represents an alkyl radical containing 1 to 6 carbon atoms or an alkenyl radical containing 2 to 6 carbon atoms and $R_1$ represents an alkyl radical containing 1 to 4 carbon atoms, characterised in that cupric chloride, in the presence of lithium chloride, is reacted in a basic polar aprotic solvent at a temperature of between 15 and 50 °C, with a product of the general formula :

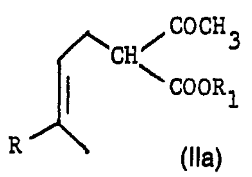

(IIa)

or

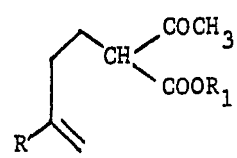

(IIb)

in which R and $R_1$ are defined as above.

2. A process according to Claim 1, characterised in that the basic polar aprotic solvent is chosen from among N-methylpyrrolidone, dimethylformamide, dimethylacetamide, tetramethylurea and hexamethylphosphotriamide.

3. Use of a product obtained according to the process of Claim 1 for the preparation of an ethylenic ketone of the general formula :

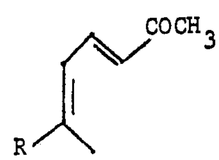

in which R is defined as in Claim 1, characterised in that a product obtained according to the process of Claim 1 is subjected to the action of lithium chloride in the presence of water or of a mineral acid, in a basic polar aprotic solvent at a temperature of about 100 °C, to give an α-chloro ketone of the general formula :

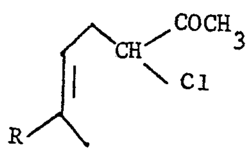

or

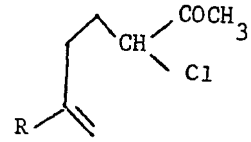

in which R is defined as in Claim 1, which compound is treated with lithium chloride in a basic polar aprotic solvent at a temperature of between 80 and 160 °C, if appropriate in the presence of a highly basic tertiary amine such as 2,4,6-trimethyl-pyridine or ethyldicyclohexylamine, to give the ethylenic ketone, which is isolated.

4. Use of a product obtained according to the process of Claim 1 for the preparation of an ethylenic ketone of the general formula :

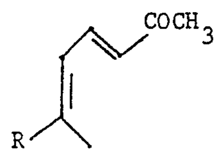

in which R is defined as in Claim 1, characterised in that a product obtained according to the process of Claim 1 is treated with the lithium chloride/mineral acid/tertiary amine system in a basic polar aprotic solvent at a temperature of between 80 and 160 °C, and the product obtained is isolated.

5. Use according to Claim 4 or 5, characterised in that the basic polar aprotic solvent is chosen from among N-methylpyrrolidone, dimethylformamide, dimethylacetamide, tetramethylurea or hexamethylphosphotriamide.

**Ansprüche** (für die Vertragsstaaten : BE, CH, DE, FR, GB, IT, LI, LU, NL, SE)

1. Ein chlorierter β-Ketoester, dadurch gekennzeichnet, daß er der allgemeinen Formel

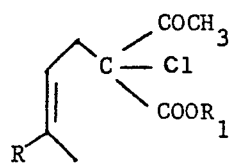

oder

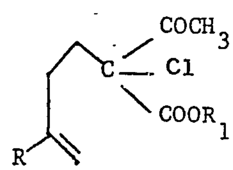

(Ia) (Ib)

entspricht,
worin R einen Alkylrest mit 1 bis 6 Kohlenstoffatomen oder einen Alkenylrest mit 2 bis 6 Kohlenstoffatomen bedeutet und $R_1$ einen Alkylrest mit 1 bis 4 Kohlenstoffatomen darstellt.

2. Ein Verfahren zur Herstellung eines Produkts gemäß Anspruch 1, dadurch gekennzeichnet, daß man Kupferchlorid in Gegenwart von Lithiumchlorid in einem aprotischen, polaren, basischen Lösungsmittel bei einer Temperatur zwischen 15 und 50 °C auf ein Produkt der allgemeinen Formel

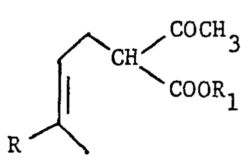

oder

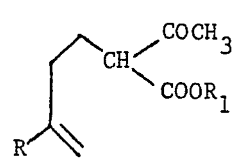

(IIa) (IIb)

worin R und $R_1$ wie in Anspruch 1 definiert sind, einwirken läßt.

3. Ein Verfahren gemäß Anspruch 1, dadurch gekennzeichnet, daß das aprotische polare basische Lösungsmittel ausgewählt ist unter N-Methylpyrrolidon, Dimethylformamid, Dimethylacetamid, Tetramethylharnstoff und Hexamethylphosphortriamid.

4. Verwendung eines Produkts gemäß Anspruch 1 zur Herstellung eines äthylenischen Ketons der allgemeinen Formel

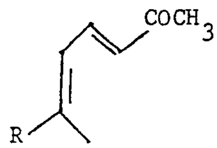

worin R wie in Anspruch 1 definiert ist, dadurch gekennzeichnet, daß ein Produkt gemäß Anspruch 1 der Einwirkung von Lithiumchlorid in Gegenwart von Wasser oder einer Mineralsäure in einem aprotischen, polaren, basischen Lösungsmittel bei einer Temperatur nahe bei 100 °C unterworfen wird, um ein α-Chlorketon der allgemeinen Formel

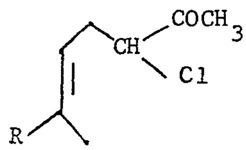

oder

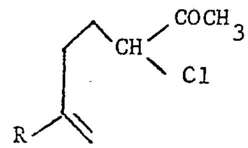

worin R wie in Anspruch 1 definiert ist, zu ergeben, das mit Lithiumchlorid in einem aprotischen polaren basischen Lösungsmittel bei einer Temperatur zwischen 80 und 160 °C, gegebenenfalls in Gegenwart eines sehr basischen tertiären Amins, wie 2,4,6-Trimethylpyridin oder Äthyldicyclohexylamin, behandelt wird, um das äthylenische Keton zu ergeben, das isoliert wird.

5. Verwendung eines Produkts gemäß Anspruch 1 zur Herstellung eines äthylenischen Ketons der allgemeinen Formel

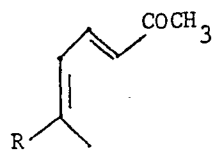

worin R wie in Anspruch 1 definiert ist, dadurch gekennzeichnet, daß man ein Produkt gemäß Anspruch 1 mit dem System Lithiumchlorid-Mineralsäure-tertiäres Amin in einem aprotischen polaren basischen Lösungsmittel bei einer Temperatur zwischen 80 °C und 160 °C behandelt und das erhaltene Produkt isoliert.

6. Verfahren gemäß Anspruch 4 oder 5, dadurch gekennzeichnet, daß aprotische, polare, basische Lösungsmittel ausgewählt ist unter N-Methylpyrrolidon, Dimethylformamid, Dimethylacetamid, Tetramethylharnstoff oder Hexamethylphosphortriamid.

**Ansprüche** (für den Vertragsstaat AT)

1. Verfahren zur Herstellung eines neuen chlorierten β-Ketoesters der allgemeinen Formel

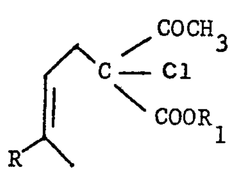

(Ia)

oder

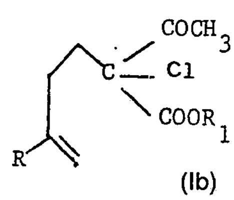

(Ib)

in welcher R einen Alkylrest mit 1 bis 6 Kohlenstoffatomen oder einen Alkenylrest mit 2 bis 6 Kohlenstoffatomen darstellt und $R_1$ einen Alkylrest mit 1 bis 4 Kohlenstoffatomen darstellt, dadurch gekennzeichnet, daß man Kupfer(II)-chlorid in Gegenwart von Lithiumchlorid in einem basischen, aprotischen, polaren Lösungsmittel bei einer Temperatur zwischen 15 und 50 °C auf eine Verbindung der allgemeinen Formel

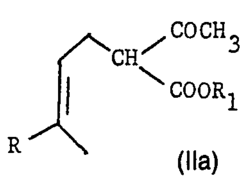

(IIa)

oder

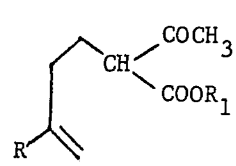

(IIb)

in welcher R und $R_1$ die obige Bedeutung haben, einwirken läßt.

2. Verfahren nach Anspruch 1, dadurch gekennzeichnet, daß das basische, aprotische, polare Lösungsmittel ausgewählt ist aus N-Methylpyrrolidon, Dimethylformamid, Dimethylacetamid, Tetramethylcarbamid und Hexamethylphosphortriamid.

3. Verwendung einer nach Anspruch 1 erhaltenen Verbindung zur Herstellung eines äthylenischen Ketons der allgemeinen Formel :

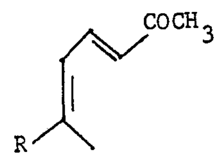

in welcher R die in Anspruch 1 angegebene Bedeutung hat, dadurch gekennzeichnet, daß man eine nach dem Verfahren des Anspruchs 1 erhaltene Verbindung der Einwirkung von Lithiumchlorid in Gegenwart von Wasser oder einer anorganischen Säure in einem basischen aprotischen polaren Lösungsmittel bei einer Temperatur nahe 100 °C unterwirft unter Bildung eines α-Chlorketons der allgemeinen Formel

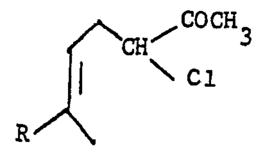

oder

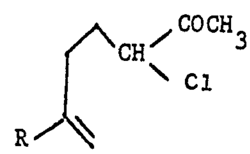

in welcher R die in Anspruch 1 angegebene Bedeutung hat, welches mit Lithiumchlorid in einem basischen aprotischen polaren Lösungsmittel bei einer Temperatur zwischen 80 und 160 °C, gegebenenfalls in Gegenwart eines sehr basischen tertiären Amins wie 2,4,6-Trimethyl-pyridin oder Äthyldicyclohexylamin, unter Bildung des äthylenischen Ketons, das isoliert wird, behandelt wird.

4. Verwendung einer nach Anspruch 1 erhaltenen Verbindung zur Herstellung eines äthylenischen Ketons der allgemeinen Formel :

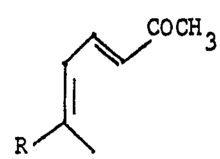

in welcher R die in Anspruch 1 angegebene Bedeutung hat, dadurch gekennzeichnet, daß man eine nach dem Verfahren des Anspruchs 1 erhaltene Verbindung mit dem System Lithiumchlorid/anorganische Säure/tertiäres Amin in einem basischen aprotischen polaren Lösungsmittel bei einer Temperatur zwischen 80 und 160 °C behandelt und das erhaltene Produkt isoliert.

5. Verwendung nach Anspruch 4 oder 5, dadurch gekennzeichnet, daß das basische aprotische polare Lösungsmittel ausgewählt ist aus N-Methylpyrrolidon, Dimethylformamid, Dimethylacetamid, Tetramethylcarbamid oder Hexamethylphosphortriamid.